(19) Europäisches Patentamt
European Patent Office
Office européen des brevets

(11) **EP 4 534 004 A1**

(12) **EUROPEAN PATENT APPLICATION**

(43) Date of publication:
**09.04.2025 Bulletin 2025/15**

(21) Application number: **23201531.3**

(22) Date of filing: **04.10.2023**

(51) International Patent Classification (IPC):
***A61B 5/024*** *(2006.01)*    ***A61B 5/1455*** *(2006.01)*
***A61B 5/00*** *(2006.01)*

(52) Cooperative Patent Classification (CPC):
**A61B 5/14551; A61B 5/02416;** A61B 5/7221;
A61B 2560/0209

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **SCHWENK, Marcus**
**5656AG Eindhoven (NL)**
• **HUCK, Salina Marie**
**5656AG Eindhoven (NL)**

(74) Representative: **Philips Intellectual Property &
Standards
High Tech Campus 52
5656 AG Eindhoven (NL)**

(54) **DEVICE, SYSTEM AND METHOD FOR OPERATING A PULSE OXIMETER**

(57) The present invention relates to a device, system and method for operating a pulse oximeter. The device comprises a processing unit (22) configured to determine the periodicity of at least one PPG signal, predict, based on the determined periodicity, if the at least one PPG signal is approaching one of its extreme points, and compute a vital sign from the at least two PPG signals. A control unit (23) generates a control signal that, i) when the at least one PPG signal is approaching one of its extreme points, controls the light emitter to activate light emission in the one or more wavelength ranges and/or controls the light detector to activate the light detection and/or generation of PPG signals in the one or more wavelength channels, and, ii) when the at least one PPG signal has passed the extreme point, controls the light emitter to deactivate light emission in the one or more wavelength ranges and/or controls the light detector to deactivate the light detection and/or the generation of PPG signals in the one or more wavelength channels.

100

FIG.8

EP 4 534 004 A1

## Description

FIELD OF THE INVENTION

The present invention relates to a device, system and method for operating a pulse oximeter.

BACKGROUND OF THE INVENTION

Pulse oximeters measure the oxygen saturation (also referred to as SpOz) of a patient's arterial blood by utilizing the knowledge that only the blood flow in arteries is strongly pulsatile, and the oxygenated and deoxygenated hemoglobin have markedly different light absorption spectra. A pulse oximeter works by measuring the intensity of light of different wavelength ranges that has either passed through or is reflected by a volume of tissue with good blood supply. This results in a photoplethysmography (PPG) signal per wavelength range (which may cover a single wavelength or narrow wavelength band). By determining the amplitudes of the pulsatile part of each PPG signal, the pulse oximeter can estimate the ratio of oxygenated hemoglobin to total hemoglobin in the arterial blood.

Pulse oximetry is widely used in clinical practice, as it is non-invasive, safe, quick and convenient. A pulse oximeter often takes the form of a clip or sleeve sensor that is attached to one of the patient's fingers or another part of the body with good blood supply, such as an earlobe. It contains a light emitter (also called light source), generally two (or more) light emitting elements such as light-emitting diodes (LEDs), to generate and emit light of different, predefined wavelengths, and a light detector (also called sensor) opposite of the light emitter to measure the intensity of light that has passed through the tissue. The light emitting elements (e.g. LEDs) are commonly activated briefly in rapid sequence, and the pulse oximeter uses analog and/or digital signal processing (as processing unit) to process the single light intensity signal from the light detector into wavelength-specific light intensity signal channels (also called PPG signals).

More recently, pulse oximeters have also found application in consumer-grade devices like smart watches and fitness trackers.

SUMMARY OF THE INVENTION

It is an object of the present invention to reduce the power consumption of a pulse oximeter and/or increase the accuracy and availability of vital signs computed by a pulse oximeter. It is a further object of the present invention to provide a corresponding method for operating a pulse oximeter as well as a system.

In a first aspect of the present invention a device is presented for operating a pulse oximeter comprising a light emitter configured to emit light in one or more wavelength ranges and a light detector configured to detect light in one or more wavelength channels reflected from skin of a subject or transmitted through tissue of the subject in response to irradiation by the light emitter, each wavelength channel covering one of the one or more wavelength ranges, and to generate one or more photoplethysmography, PPG, signals from the detected light in the one or more wavelength channels, the device comprising:

a processing unit configured to

- determine the periodicity of at least one PPG signal,
- predict, based on the determined periodicity, if the at least one PPG signal is approaching one of its extreme points, and
- compute a vital sign from the at least two PPG signals; and

a control unit configured to generate a control signal that,

i) when the at least one PPG signal is approaching one of its extreme points, controls the light emitter to activate light emission in the one or more wavelength ranges and/or controls the light detector to activate the light detection and/or generation of PPG signals in the one or more wavelength channels, and,

ii) when the at least one PPG signal has passed the extreme point, controls the light emitter to deactivate light emission in the one or more wavelength ranges and/or controls the light detector to deactivate the light detection and/or the generation of PPG signals in the one or more wavelength channels.

In a further aspect of the present invention a system is presented comprising:

- a pulse oximeter comprising a light emitter configured to emit light in one or more wavelength ranges and a light detector configured to detect light in one or more wavelength channels reflected from skin of a subject or transmitted through tissue of the subject in response to irradiation by the light emitter, each wavelength channel covering one of the one or more wavelength ranges, and to generate one or more photoplethysmography, PPG, signals from the detected light in the one or more wavelength channels; and
- a device as disclosed herein for operating the pulse oximeter.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea that not all parts of the PPG waves (also called PPG signals) recorded by a pulse oximeter are equally relevant to deriving a vital sign such as a blood oxygen saturation value. Generally, the PPG wave shows a rather stable periodicity, which may be determined (estimated, predicted or computed) based on the last periods. Since the periodicity is the same in all wavelength channels it is sufficient to only use one PPG signal to determine the periodicity. Based thereon, it can be predicted if a PPG signal is approaching one of its extreme points (i.e., a maximum or minimum). These extreme points of the one or more PPG signals, in particular a segment of the one or more PPG signals around the extreme points represent high-information segments of the PPG waveform, i.e., segments carrying the most relevant information with respect to the generation of vital sign(s).

According to the present invention, the one or more wavelength channels are thus only activated when the corresponding PPG waveform reaches an absolute extreme point and are deactivated in between the extreme points. Hereby, the deactivation of a wavelength channel shall be understood as deactivation of the light emission in this wavelength channel and/or as deactivation of the light detection and/or the generation of PPG signals in this wavelength channel.

The present invention thus makes use of the physiological knowledge that the change in light intensity between the end of diastole and the end of systole is rapid and contains both relevant extreme points. The lowest light absorption occurs at the end of diastole, while the greatest light absorption occurs towards the end of the systole. The light absorption in tissue is often described using a multiplicative model, i.e., the total absorbed ratio of light is equal to the product of absorptions of individual components of the absorbing value. The multiplicative model can be converted to a linear model by taking the logarithm, which turns the product of individual absorptions into a sum of the logarithms of the individual absorptions.

The proposed control leads to a considerable reduction of the power consumption of the pulse oximeter and allows a longer run-time of battery-operated pulse oximeters, which is particularly advantageous for portable/wearable pulse oximeters. Further, it may increase the accuracy and availability of the determined vital sign if only the most relevant information carried by the high-information segments of the PPG waveform is used for generating the desired vital sign, e.g. a SpO$_2$ numeric produced by a pulse oximeter.

The proposed approach may be controlled to be applied only if the subject's heart rate is within a predefined range, e.g., if the PPG periodicity is above 30 beats per minute (BPM) and below 180 BPM, which represent the conventional control pattern for patients at risk.

It shall be noted that the present invention may be used in the context of determining a vital sign (e.g. HR) from a single PPG signal using light (or, more generally, radiation) detected in a single wavelength channel, but is preferably used in the context of determining a vital sign from two (or more) PPG signals using light (or radiation) a two or more different wavelength channels (e.g. SpO$_2$).

According to a preferred embodiment the processing unit is configured to determine the stability of the periodicity of the at least one PPG signal and the control unit is configured to control the pulse oximeter to

- activate or remain in a first mode, in which the light emitter and/or the light detector are controlled according to the control signal, if the periodicity of the at least one PPG signal is stable, in particular if the determined stability is equal to or higher than a stability threshold; or
- activate or remain in a second mode, in which the light emitter continuously or periodically emits light in the one or more wavelength ranges and the light detector continuously or repeatedly detects light in one or more wavelength channels, if the periodicity of the at least one PPG signal is not stable, in particular if the determined stability is lower than the stability threshold used for determining if the pulse oximeter shall be controlled to activate or remain in the first mode or a different stability threshold.

This makes sure that only in case of a sufficient stability of the periodicity of the at least one PPG signal the wavelength channels are temporarily deactivated according to the control signal in the first mode. In case of insufficient stability, the wavelength channels are activated continuously to avoid missing any critical situation of the subject.

There are generally different options to determine the stability of the periodicity of the at least one PPG signal. According to an embodiment the processing unit is configured to determine the stability of the periodicity of the at least one PPG signal by determining the number of extrema for a measurement duration and determining a mean or average value and/or standard deviation of the number of extrema, in particular by determining a mean or average value and/or standard deviation of the number of extrema for two or more measurement periods and comparing the determined mean or average values and/or standard deviation determined for the two or more measurement periods. Generally, the heart rate of a subject changes slowly and gradually over time, which is considered (classified) as stable condition. However, a stable average heart rate may show single beats missing in a periodic pattern, which may be considered (classified) as unstable condition.

According to a further embodiment, the processing unit is configured to determine the signal quality, in particular the signal-to-noise ratio, of the at least one PPG signal and the control unit is configured to control the pulse oximeter to

- activate or remain in the first mode, if the periodicity of the at least one PPG signal is stable, in particular if the determined stability is equal to or higher than a stability threshold, and the signal quality is high, in particular if the determined signal quality is equal to or higher than a quality threshold; or
- activate or remain in a second mode, if the stability of the at least one PPG signal is not stable, in particular if the determined stability is lower than the stability threshold, and/or the signal quality is low, in particular if the determined signal quality is lower than the quality threshold used for determining if the pulse oximeter shall be controlled to activate or remain in the first mode or a different quality threshold.

According to this embodiment the signal quality is used as an additional criterium for determining if the periodicity is stable, which may improve the accuracy of the determination of the stability.

In the context of determining stability, various parameters may be set or controlled, such as the length of the measurement periods, which two or more measurement periods to compare, and the level of one or more stability thresholds for the stability criteria.

The processing unit may be configured to generate the control signal that,

i) when the at least one PPG signal is approaching one of its extreme points, controls the light detector to acquire a limited number of samples, in particular one or more samples below the maximum number of samples or preferably below half of the maximum number of samples, of the one or more PPG signals around the extreme point, and,

ii) when the at least one PPG signal has passed the extreme point, controls the light detector not to acquire samples of the one or more PPG signals in the one or more wavelength channels.

Thus, the number of samples acquired and used in the different modes is controlled according to this embodiment. Reducing the number of samples that need to be acquired (and processed) will efficiently reduce power consumption of the pulse oximeter.

In another embodiment the processing unit is configured to detect if the at least one PPG signal is approaching one of its extreme points based on an evaluation of the recent signal course of the at least one PPG signal and/or the derivation or slope of the at least one PPG signal. Typically, the pulses of a PPG signal do not rapidly change completely so that the course (as well as derivation or slope) of the PPG signal can be somewhat predicted or estimated from the recent development of the course and/or derivation and/or slope of the PPG signal. This provides a simple but efficient way of detecting if the PPG signal approaches an extreme point.

In an embodiment the processing unit is configured to determine that the at least one PPG signal is approaching one of its extreme points if the absolute value of the difference between the actual signal value of the first PPG signal and the average value of the first PPG signal exceeds a value in the range of 25% to 45%, in particular that exceeds 30%, 40% or 45% of the difference between the last two extreme points or an average of the last differences between respective two consecutive extreme points. In practical implementations, values between 25% and 45% are sensible, depending on the start-up time of the wavelength channel. Smaller values than 25% may allow only short deactivation periods of the wavelength channels and may result in little power savings only. Values larger than 45% may result in pulses being missed due to intrinsic variability of the extreme point values. Smaller values may reduce both the power savings and the risk of missing pulses. Larger values may maximize power savings but may also increase the chance of missing pulses.

In another embodiment the processing unit is configured to determine that the at least one PPG signal is approaching one of its extreme points if the derivation or slope falls below a value in the range of 25% to 50%, in particular that falls below 50% or 40% or 30% of the derivative of the last extreme point or an average of derivates of the last two or more extreme points. As is generally known, a derivative approaches zero when a function approaches an extreme point. A simple implementation would be activating the wavelength channels when the numeric/estimated derivative of the PPG wave is less than 20% ... 50% of the last extreme value of the derivative. The best value may depend on the start-up delay of the wavelength channels (e.g. the device may require some time for starting up, which may be a period in the range of some ms (milliseconds) or some ten ms up to one second).

According to an embodiment the processing unit is configured to detect if the at least one PPG signal is approaching one of its extreme points based on physiological knowledge about the subject and/or the recent computation of the vital sign. For instance, the processing unit may be configured to determine that the first PPG signal is approaching one of its extreme points if a time period from the last extreme point has passed that corresponds to a value in the range of 90% to 99%, in particular 90% or 95% or 97,5%, of the time period between the last two extreme points or an average of the last multiple time periods between respective two consecutive extreme points. A valued closer to 90% may be more appropriate since some heart rate variability is generally expected. The number may also depend on the detected heart rate. For fast heart rates, the number could be closer to 99%, and it may not matter much if a pulse is missed when the heart rate is high, e.g. 120 bpm, since there will quickly be another pulse, with a HR of 120 bpm in about half a second.

According to another embodiment the processing unit is configured to determine that the at least one PPG signal has passed the extreme point if the absolute value of the difference between the actual signal value of the at least one PPG signal and the average value of the at least one PPG signal falls below a value in the range of 25% to 45%, in particular that falls below 30%, 40% or 45% of the difference between the last two extreme points or an average of the last multiple differences between respective two consecutive extreme points. A value of or around 40% may be a good starting point, but this value may be adjusted downwards if it is detected that an extreme point has been missed. This detection may be possible using the first, continuous PPG signal. The value may also be adjusted upwards if the history shows reliable capture of the extreme points.

According to another embodiment the processing unit is configured to determine that the at least one PPG signal has passed the extreme point after a time period from reaching the extreme point that corresponds to the time period from activating the light emission in one or more wavelength ranges up to reaching the extreme point. This provides a simple but efficient way of controlling the deactivation of the wavelength channels.

In another embodiment the control unit is configured to activate and deactivate light emission in the one or more wavelength ranges for a time duration in the range up to 30% of the time period between the last two extreme points or an average of the last multiple time periods between respective two consecutive extreme points. 30% activation would correspond to a power usage for driving the LEDs of 30% compared to running both wavelength channels continuously (in case of two wavelength channels in total). Thus, considerable power savings can be obtained.

In another embodiment the control unit is configured to control the light detector to activate and deactivate the light detection and/or generation of PPG signals in the one or more wavelength channels simultaneously or substantially simultaneously with activation and deactivation of the light emitter, in particular to activate and deactivate signal acquisition circuitry and/or signal processing circuitry of the light detector. This may further reduce the total power consumption.

In another embodiment the processing unit is configured to generate the vital signal, in particular blood oxygen saturation or heart rate or a modification of hemoglobin, from the extreme values of the at least two PPG signals. Although heart rate can be obtained from a single PPG signal only, the present invention may be applied in such a scenario as well if e.g. heart rate shall be continuously generated and a second vital sign that requires one or more PPG signals shall only be generated intermittently or from time to time. If more than two wavelengths are used, the invention may be used to distinguish one or more modifications of hemoglobin, in particular oxyhemoglobin, deoxyhemoglobin, methemoglobin (that forms with nitrites, certain toxic substances, and certain pharmaceuticals) and/or carboxyhemoglobin (that forms with carbon monoxide).

The pulse oximeter may generally be controlled to operate with only one activation/deactivation cycle per pulse instead of two activation/deactivation cycles per pulse if it captures both extreme points within this one cycle.

The disclosed system may comprise the pulse oximeter and the device for operating the pulse oximeter as separate entities. For instance, the device may be implemented as processor, e.g. as part of a computer or a patient monitor. Alternatively, the device may be part of the pulse oximeter, e.g. as a built-in processor, so that the pulse oximeter is a standalone/wearable device, e.g. in the form of a clip that can be mounted to the finger, ear lobe or nasal alar or in the form of a body-mountable sensor that can be attached to the body, e.g. by an adhesive or a belt.

BRIEF DESCRIPTION OF THE DRAWINGS

These and other aspects of the invention will be apparent from and elucidated with reference to the embodiment(s) described hereinafter. In the following drawings

Fig. 1 shows a diagram of a two PPG signals for two continuously active wavelength channels.
Fig. 2 shows a diagram of the intensities of two wavelength channels illustrated in Fig. 1 over each other as data points.
Fig. 3 shows a diagram of two PPG signals for two partly deactivated wavelength channels.
Fig. 4 shows a diagram of the intensities of two wavelength channels illustrated in Fig. 3 over each other as data points.
Fig. 5 shows a schematic diagram of an embodiment of a system according to the present invention.
Fig. 6 shows a schematic diagram of an exemplary implementation of a device according to the present invention.
Fig. 7 shows a schematic diagram of an embodiment of a device according to the present invention.
Fig. 8 shows a schematic diagram of an embodiment of a method according to the present invention.
Fig. 9 shows a schematic diagram of an exemplary implementation of a system according to the present invention.

## DETAILED DESCRIPTION OF EMBODIMENTS

Known pulse oximeters commonly record and process all of their wavelength channels continuously, digitally sampling each channel with a constant, uniform sampling rate. This results in a set of PPG waves (PPG signals) that are easy to process using common signal processing techniques like linear digital filters which presuppose uniformly sampled signals.

This convenience is paid for by spending energy to acquire redundant information. This expenditure of energy reduces the run-time of battery-powered pulse oximeters. Pulse oximeters may also have a limit on the average power they expose to the patient (or, more generally, the subject) to in the form of light and heat. In general, spending more power in the form of higher light intensities, higher power draw of operational amplifiers, higher sampling rates or higher sampling resolution results in a more accurate measurement with a higher signal-to-noise ratio (SNR). Considering this, spending the same amount of power acquiring both information-rich and information-poor segments of the PPG wave appears inefficient. Allocating more of the average power budget to acquire the highly interesting segments of the PPG waves with high reliability and high SNR and spending as little of the average power budget on acquiring data from segments with low information content may improve the overall quality of the desired vital sign derived by the pulse oximeter, e.g. a blood oxygen saturation value.

In general, the information relevant for pulse oximetry is contained in the PPG wave segments where the PPG wave is close to its absolute extreme points. Segments where the PPG wave undergoes rapid changes may also be considered interesting, depending on the algorithm used by the pulse oximeter. The PPG signals across different wavelength channels are highly correlated, so the knowledge of a single wavelength channel allows conclusions about the likely waveforms in all other channels.

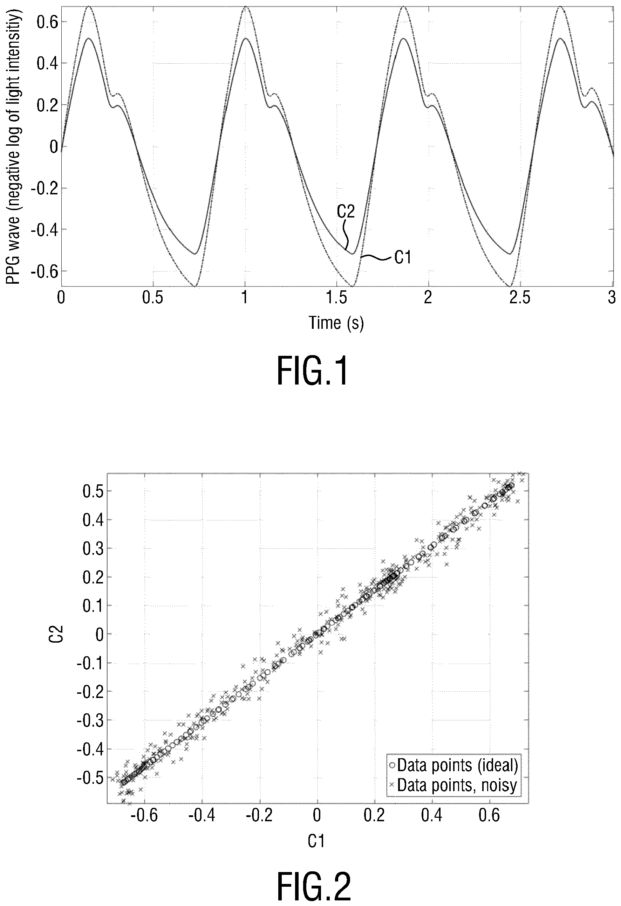

Fig. 1 shows a diagram of a simulation of a two-channel photoplethysmogram, where the two channels C1 and C2 are continuously active. Preferably, the two-channel photoplethysmogram is sampled with maximum sampling rate to acquire a maximum number of samples is used for the subsequent evaluation. Fig. 2 shows a diagram with a different perspective, dropping the time series information and plotting light intensities of one channel C1 over the light intensities of the other channel C2 as data points. Intuitively, the ratio of magnitudes of variation of the two channels is found as the slope of the point of clouds. The diagram also shows that for given noise levels, the points near the ends of the cloud have a higher signal-to-noise ratio than the points near the middle of the cloud. In a perfect, noise-less situation, any choice of data points would allow accurate calculation of the slope.

The present invention is based on the insight that not all parts of the PPG waves recorded by a pulse oximeter are equally relevant to deriving a blood oxygen saturation value. If the PPG waveform is estimated (or predicted) based on the generally rather stable periodicity, the wavelength channels only need to be active during high-information segments of the PPG waveform and can be powered down in less relevant segments of the PPG waveform.

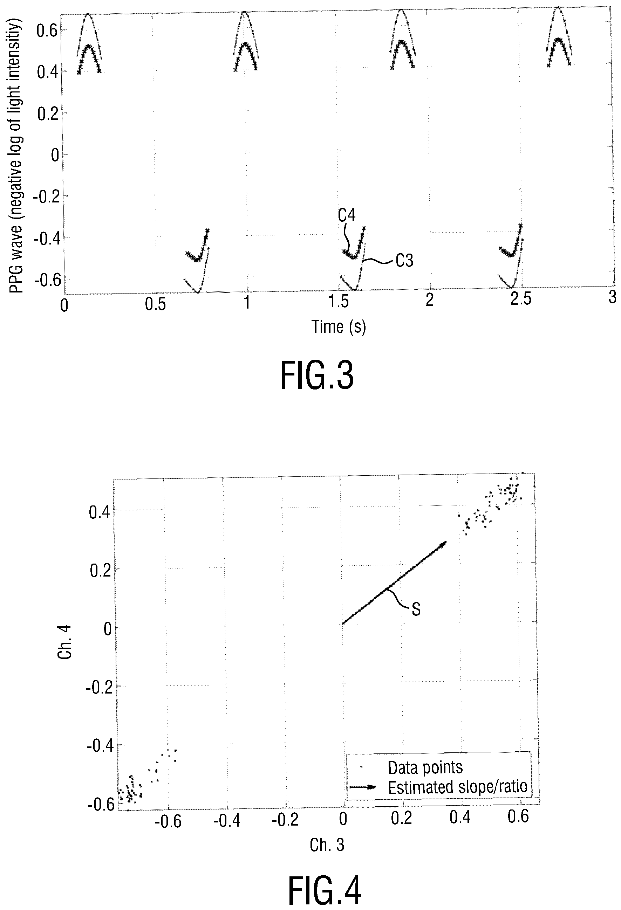

This is illustrated in Figs. 3 and 4. Fig. 3 shows a diagram, similar to Fig. 1, of a simulation of a two-channel photoplethysmogram, where both channels C3 and C4 are only active around the extreme points of the PPG wave. Preferably, only a lower number (i.e. lower than the maximum number) of samples is acquired from the two-channel photoplethysmogram so that only a few samples around the extrema are sampled and used for the subsequent evaluation. Fig. 4 shows a diagram, similar to the diagram of Fig. 2, that plots light intensities of the channel C3 over the light intensities of the channel C4 as data points. Further, it includes an indication of the slope S estimated from the reduced number of data points.

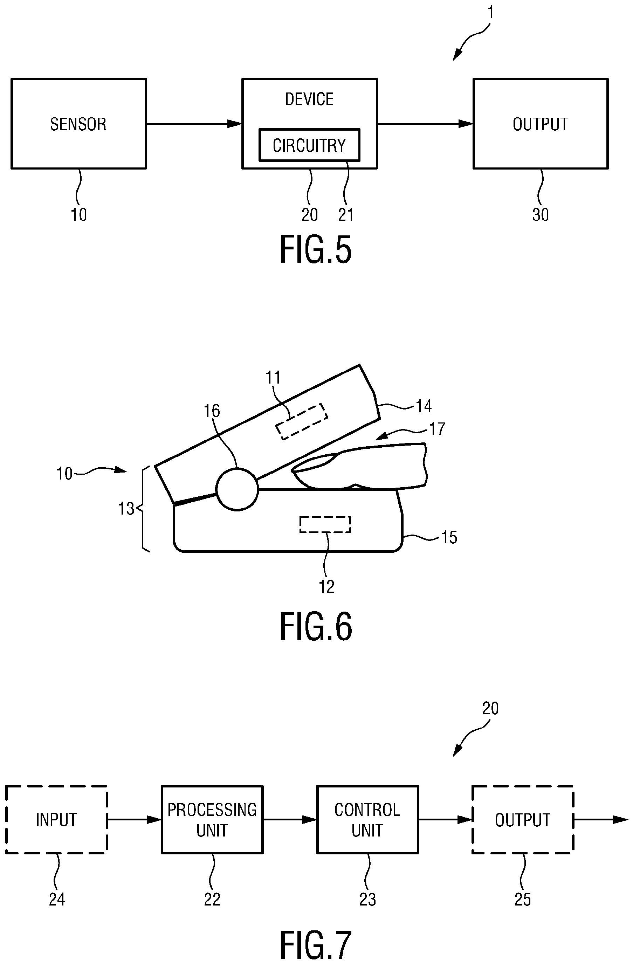

Fig. 5 shows a schematic diagram of an embodiment of a system 1 for determining a vital sign of a subject, e.g. for determining blood oxygen saturation ($SpO_2$) of a subject. The system 1 comprises a sensor 10, in particular a pulse oximeter, including a light emitter 11 and a light detector 12 (as shown in Fig. 6) configured to acquire photoplethysmography (PPG) signals at a measurement location of the subject. The system 1 further comprises a device 20 for operating the sensor 10 as will be explained in more detail below.

Optionally, the system 1 may further comprise an output interface 30 configured to issue the determined vital sign. The optional output interface 30 may generally be any means that outputs the determined vital sign in visual or audible form, e.g. in text form, as image or diagram, as sound or spoken words, etc. For instance, the output interface 30 may be a display, a loudspeaker, a touchscreen, a computer monitor, the screen of a smartphone or tablet, etc. In an embodiment, the output interface 30 may be a separate device or may be combined with the sensor 10 or the device 20, e.g. may be integrated into the housing of the sensor 10, for instance in the form of a display within or on top of a surface of the housing.

The device 20 may be implemented by respective units or circuitry 21, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 20, which may execute a computer program that may be stored in a memory that is accessed by the processor.

Fig. 6 shows a side view of a pulse oximeter 10 in an open state. In this exemplary embodiment the pulse oximeter 10 is configured substantially in the same manner as a conventional pulse oximeter in the form of a finger clip that can be clipped at the subject's finger. The pulse oximeter 10 has a housing 13 formed by two housing portions 14, 15 that are hinged together by a swivel joint 16. Between the two housing portions 14, 15 a measurement location 17 (also called measurement area) is formed.

Light is emitted by the light emitter 11 towards the measurement location, which is the part (in this example the fingertip) of the subject's anatomical object (in this example the finger) that is arranged in the measurement area 17. The light detector 12 detects light that has, after emission from the light emitter 11, transmitted through the subject's tissue (i.e., the fingertip) at the measurement location 17.

In other embodiments the sensor 10 may be configured for arrangement at other anatomical objects, such as the ear lobe or the nasal alar. It may be configured as clip for clipping it to the anatomical object, as shown in Fig. 6, or as wearable, e.g. in the form of or integrated into a smartwatch, belt or other means that can be mounted (e.g. taped) to the subject's body. Particularly in sensors formed as wearables, the light detector 12 may be configured to detect light that has, after emission from the light emitter 11, been reflected from the subject's tissue, in particular the subject's skin. Thus, both the light emitter 41 and the light detector 42 may be arranged within the same housing portion and may be a placed next to each other.

The light emitter 11 is configured to emit light in one or more different wavelength ranges, e.g. in two (or more) different wavelength ranges such as near-infrared light and red light. It may comprise two separate LEDs that emit the respective light in the two (or more) wavelength channels, either simultaneously or alternately.

The light detector 12 is configured to detect light in one or more wavelength channels (preferably two or more different wavelength channels) reflected from skin of the subject or transmitted through tissue of the subject in response to irradiation by the light emitter 11. Each wavelength channel covers a separate wavelength range, i.e., in case of two or more wavelength channels each preferably covers a different wavelength range. The light detector 12 may comprise a single photosensor unit that is sensitive to light in a broad wavelength range or only in the wavelength ranges of interest in which light is emitted by the light emitter (e.g. red light and near-infrared light). In other embodiments separate photodiodes may be used which are each sensitive for light in different wavelength channels (e.g. one photodiode for red light and another photodiode for near-infrared light).

From the detected light in the respective wavelength channels two (or more) PPG signals are generated. A PPG signal represents the intensity of the detected light over time in a wavelength channel of interest. For instance, two PPG signals in the red and near-infrared wavelength channels may be derived from the detected light. This may be done by the light detector 12, which may comprise processing circuitry to process the detected light accordingly. In other embodiments this processing may be done in the device 20.

The device 20 is preferably combined with the pulse oximeter 10, e.g. is integrated into the housing 13 of the pulse oximeter 10. In other embodiments the device 20 may be a separate device. Generally, the PPG signals acquired by the pulse oximeter 10 may be provided to the device 20 through a wired or wireless connection.

Fig. 7 shows a schematic diagram of an embodiment of a device 20 for operating the pulse oximeter according to the present invention. In this embodiment, the device 20 comprises a processing unit 22 that determines the periodicity of at least one PPG signal, predicts, based on the determined periodicity, if the at least one PPG signal is approaching one of its extreme points, and computes a vital sign from the at least two PPG signals. A control unit 23 generates a control signal for controlling the light emitter 11 and/or the light detector 12.

Optionally, an input 24 may be provided to obtain the PPG signals from the sensor 10. The input 21 may be directly coupled or connected to the sensor 10 or may obtain (i.e. retrieve or receive) the PPG signals from a storage, buffer, network, or bus, etc. The input 24 may thus e.g. be (wired or wireless) communication interface or data interface, such as a Bluetooth interface, Wi-Fi interface, LAN interface, HDMI interface, direct cable connection, or any other suitable interface allowing signal transfer to the device 20.

Further, the device 20 may optionally comprise an output 25 configured to output the determined control signal and the determined vital sign. The output 25 may generally be any interface that provides the determined control signal to the light emitter 11 and/or the light detector 12. Further, it provides the determined vital sign, e.g. transmits it to another device or provides it for retrieval by another device (e.g. a smartphone, computer, tablet, etc.), or simply outputs it to a display, e.g. the output interface 30. It may thus generally be any (wired or wireless) communication or data interface.

The processing unit 22 and the control unit 23 may be any kind of means configured to process the PPG signals and determine the control signal and the desired vital sign. They may be implemented in software and/or hardware, e.g. as common or separate programmed processor or computer or app on a user device such as a smartphone, smartwatch, tablet, laptop, PC, workstation, etc.

The processing unit 22 and the control unit 23 (and, optionally an input 24 and an output 25) may together form the circuitry 21 of the device 20.

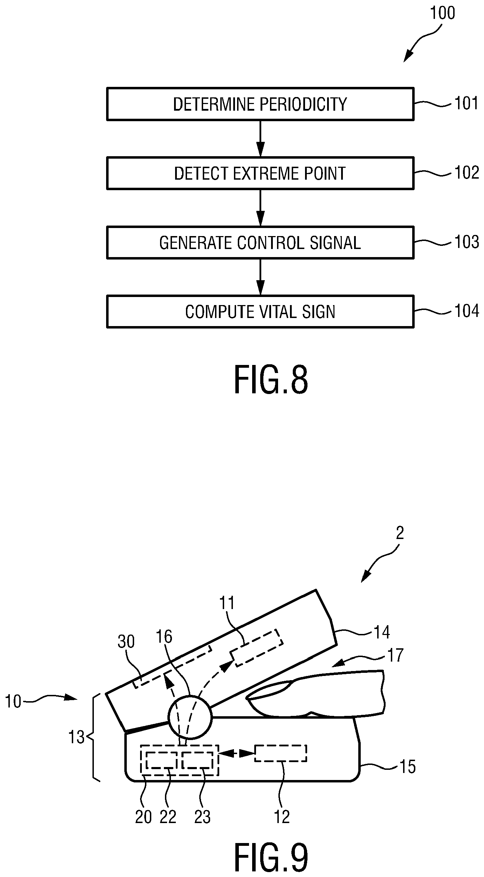

Fig. 8 shows a schematic diagram of an embodiment of a method 100 for operating the pulse oximeter according to the present invention. The steps of the method may be carried out by the device 20, wherein the main steps of the method are carried out by the processing unit 22 and the control unit 23. The method may e.g. be implemented as computer program running on a computer or processor.

In a first step 101, the periodicity of at least one PPG signal is determined. In a second step 102, based on the determined periodicity, it is predicted if the at least one PPG signal is approaching one of its extreme points. In a third step 103, a control signal is generated for activating and deactivating the wavelength channels based on the prediction in step 102. In a fourth step 104, a vital sign is computed from the at least two PPG signals. For instance, the ratio of the magnitudes of variability of each PPG signal due to the pulsation of arterial blood may be computed, which ratio is then converted to an estimate of the blood oxygen saturation in a known manner, e.g. by use of a calibration using a calibration table or function.

The control signal is configured to control the light emitter 11 and/or the light detector 12. Preferably, at least the light emitter 11 is controlled since larger power savings can be achieved by only temporarily activating the wavelength channels.

When the at least one PPG signal is approaching one of its extreme points, the control signal controls the light emitter to activate light emission in the one or more wavelength ranges (preferably in all wavelength ranges) and/or the light detector to activate the light detection and/or generation of PPG signals in the one or more wavelength channels (preferably in all wavelength channels). When the at least one PPG signal has passed the extreme point, the control signal controls the light emitter to deactivate light emission in the one or more wavelength ranges (preferably in all wavelength ranges) and/or controls the light detector to deactivate the light detection and/or the generation of PPG signals in the one or more wavelength channels (preferably in all wavelength channels).

In this way, a considerable power saving can be achieved so that a battery-operated pulse oximeter has a longer operation time. Further, since in the wavelength channels only the information-rich segments of the PPG signals are evaluated which improves reliability and accuracy of the determined vital sign.

Thus, as explained above, an embodiment of the disclosed system comprises a pulse oximeter with two (or more) wavelength channels. The wavelength channels are only activated when the software of the pulse oximeter determines that the PPG waveform is likely to be near one of its absolute extreme points.

This decision may be made using already acquired data from the wavelength channels, which allows to predict the periodicity of the wavelength channels and when the next extreme point is approached so that the light emitter and/or light detector may be activated right before the next extreme point is reached. In addition, previously derived pulse rate values and/or physiological knowledge such as the ranges for durations of systole and diastole may be used to determine if the PPG waveform is deemed to approach or to be close to one the extreme points. The deactivation is happening after the extreme point has passed.

Deactivation of a channel may include powering down the light source of this channel but can also include powering down analog signal acquisition circuitry and an analog-to-digital converter (ADC) used for digitally sampling the light intensity values, in particular if the design uses more than one ADC for digitally sampling light intensity values.

In an embodiment the pulse oximeter also recovers the amplitude ratio from both sampled channels. In a simple embodiment this is done by computing the ratio of scalar products of time indexes Nb, at which values for both samples are available, after subtracting the mean value of each channel or another estimate of the expected value:

$$Ratio\left(\frac{f_2}{f_1}\right) = \frac{\langle f_{2,Nb}, f_{1,Nb}\rangle}{\langle f_{1,Nb}, f_{1,Nb}\rangle}$$

However, it should be ensured by the pulse oximeter that the correlation of the signal in the two channels is maintained, and no asymmetric delays or phase shifts are introduced. This method does not require estimation of any unavailable sample values, nor does it require uniform sampling or even any particular ordering of the data points.

Fig. 9 shows a schematic diagram of an exemplary implementation of a system 2 of the present invention. In this implementation, the device 20, in particular the processing unit 22 and the control unit 23, is integrated into the pulse oximeter 10. Further, a display 30 is arranged within the housing portion 14, e.g. to display the computed vital sign. In another embodiment, the elements of the system may be distributed or implemented separately, e.g. as sensor connected to a pulse oximeter (e.g. via cable or wireless) and (patient) monitor.

In an embodiment, the stability of the periodicity of the at least one PPG signal is determined, e.g. by determining if the time periods between two subsequent maxima or minima are constant or varying. If the periodicity is determined to be stable, e.g. if the determined stability is equal to or higher than a stability threshold, the pulse oximeter is controlled to activate or remain in a first mode (also called "reduced sample mode"), in which the light emitter and/or the light detector are controlled according to the control signal. In an embodiment, the photoplethysmogram may be tracked continuously and individual samples (between the extrema) may be skipped.

If the periodicity is determined to be unstable, e.g. if the determined stability is lower than a stability threshold, the pulse oximeter is controlled to activate or remain in a second mode (also called "full sampling mode"), in which the light emitter continuously or periodically emits light in the one (or more different) wavelength range(s) and the light detector continuously or repeatedly detects light in one (or more different) wavelength channel(s).

The stability threshold may be a metric regarding the length and/or changes of the length of the periodicity over time. It may be predetermined and fixed or it may be set and varied by a user.

In an embodiment the number of extrema for a measurement duration and a mean or average value and/or standard deviation of the number of extrema may be determined for determining the stability of the periodicity. For instance, a mean or average value and/or a standard deviation of the number of extrema for two or more measurement periods may be determined and compared.

In another embodiment, the signal quality, in particular the signal-to-noise ratio (SNR), of the at least one PPG signal may be used in addition to determine if the pulse oximeter shall be switched into the first mode ("reduced sampling mode") or into the second mode ("full sampling mode"). The signal quality may be determined from the power spectrum of the photoplethysmogram waveforms. If the signal quality is high, in particular if the determined signal quality is equal to or higher than a quality threshold, the first mode may be used, and if the signal quality is low, in particular if the determined signal quality is lower than the quality threshold used for determining if the pulse oximeter shall be controlled to activate or remain in the first mode or a different quality threshold.

The quality threshold may be a metric regarding the signal quality, e.g. an SNR threshold. It may be predetermined and fixed or it may be set and varied by a user.

In a practical implementation, the as metric for the stability of periodicity, the periodicity of the photoplethysmogram signal may be determined in terms of beats per minute over a predefined period of time and its mean and standard deviation may be measured. As metric for the signal quality, e.g. the SNR, the power of the base signal with the determined periodicity may be determined. Further, the power of non-related frequency components may be determined, and a signal-to-noise power ratio may be qualified.

Thresholds for both stability and signal quality may be determined in a way that, when reduced sampling applies, a vital signs determination algorithm, e.g. an $SpO_2$ algorithm, stays well at acceptable accuracy, e.g. in terms of $SpO_2\,A_{rms}$, which is an accuracy measure. "A" stands for "accuracy" and "rms" for root mean square

$$A_{\mathrm{rms}} = \sqrt{\frac{\sum_{i=1}^{n}\left(SpO_{2i} - S_{Ri}\right)^2}{n}}$$

From the assumed stable periodicity the position in time of the next minimum and maximum of the photoplethysmogram may be inferred. It schedules to sample only several samples around these extrema, e.g.

at minimum three samples per extremum. Having a minimum of three sample point allows exact interpolation of the actual extremum. The amplitude of the actual extremum is used for subsequent $SpO_2$ determination. Further, interpolation from several points allows to estimate harmonics to the base periodicity, which is used in current $SpO_2$ algorithms.

From interpolated extrema new information for the periodicity can be derived, and from this information, the next set of maximum and minimum is derived. Clinical data have shown that removing samples and replacing them from interpolation data is possible without losing accuracy for selected signal conditions while lowering power consumption significantly down to 10-25 %. The present invention can thus preferably be used for low power applications.

There are different options to determine if a PPG signal is approaching one of its extreme points: According to one option it is determined if the absolute value of the difference between the actual signal value of the first PPG signal and the average value of the first PPG signal exceeds a value in the range of 25% to 45% (e.g. exceeds 30%, 40% or 45% or any other value in this range) of the difference between the last two extreme points or an average of the last differences between respective two consecutive extreme points. According to another option it is determined if the derivation or slope falls below a value in the range of 25% to 50% (e.g. falls below 50% or 40% or 30% or any other value in this range) of the derivative of the last extreme point or an average of derivates of the last two or more extreme points. According to another option it is determined if a time period from the last extreme point has passed that corresponds to a value in the range of 90% to 99% (e.g. 90% or 95% or 97,5% or any other value in this range) of the time period between the last two extreme points or an average of the last multiple time periods between respective two consecutive extreme points.

There are also different options to determine if a PPG signal has passed one of its extreme points: According to one option it is determined if the absolute value of the difference between the actual signal value of the PPG signal and the average value of the PPG signal falls below a value in the range of 25% to 45% (falls below 30%, 40% or 45% or any other value in this range) of the difference between the last two extreme points or an average of the last multiple differences between respective two consecutive extreme points. According to another option it is determined if a time period from reaching the extreme point that corresponds to the time period from activating the light emission in one or more of the other wavelength ranges up to reaching the extreme point has passed.

In an embodiment more than two wavelength channels may be used. An embodiment with more than two wavelength channels could only use one continuously sampled wavelength channel.

According to embodiments of the present invention non-uniformly sampled PPG data are processed and used to determine the ratio of variability magnitudes. The approach using scalar products described above may work for conditions with good PPG signal-to-noise ratios. Robust methods, for example employing median filters, may be used to increase the resistance to outliers.

According to other embodiments of the present invention, the two-channel pulse oximeter can shut down the signal acquisition of the two channels for large parts of the cardiac cycle. This reduces the power consumption of the pulse oximeter. More specifically, it reduces the power dissipation in the sensor part of the pulse oximeter that is applied to the patient. The reduced power consumption translates into a longer battery run-time of battery-powered devices. It also means that the available power dissipation budget is allocated efficiently to record the most information-rich segments of the PPG. Power dissipation in the sensor is limited by thermal limitations and other requirements given by applicable standards for pulse oximeters.

Thus, the present invention adds value to a pulse oximeter by allowing it to run longer on batteries, or by reaching the same battery run-time using a smaller and lighter battery. Additionally, it may improve the accuracy and availability of the desired vital sign, in particular a $SpO_2$ value, derived by the pulse oximeter through more efficient allocation of the power available for PPG measurements.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

**1.** Device (20) for operating a pulse oximeter (10) comprising a light emitter (11) configured to emit light in one or more wavelength ranges and a light detector (12) configured to detect light in one or more wavelength channels reflected from skin of a subject or transmitted through tissue of the subject in response to irradiation by the light emitter, each wavelength channel covering one of the one or more wavelength ranges, and to generate one or more photoplethysmography, PPG, signals from the detected light in the one or more wavelength channels, the device comprising:

a processing unit (22) configured to

- determine the periodicity of at least one PPG signal,
- predict, based on the determined periodicity, if the at least one PPG signal is approaching one of its extreme points, and
- compute a vital sign from the at least two PPG signals; and

a control unit (23) configured to generate a control signal that,

i) when the at least one PPG signal is approaching one of its extreme points, controls the light emitter to activate light emission in the one or more wavelength ranges and/or controls the light detector to activate the light detection and/or generation of PPG signals in the one or more wavelength channels, and,
ii) when the at least one PPG signal has passed the extreme point, controls the light emitter to deactivate light emission in the one or more wavelength ranges and/or controls the light detector to deactivate the light detection and/or the generation of PPG signals in the one or more wavelength channels.

**2.** Device according to claim 1,

wherein the processing unit (22) is configured to determine the stability of the periodicity of the at least one PPG signal; and
wherein the control unit (23) is configured to control the pulse oximeter (10) to

- activate or remain in a first mode, in which the light emitter and/or the light detector are controlled according to the control signal, if the periodicity of the at least one PPG signal is stable, in particular if the determined stability is equal to or higher than a stability threshold; or
- activate or remain in a second mode, in which the light emitter continuously or periodically emits light in the one or more wavelength ranges and the light detector continuously or repeatedly detects light in one or more wavelength channels, if the periodicity of the at least one PPG signal is not stable, in particular if the determined stability is lower than the stability threshold used for determining if the pulse oximeter (10) shall be controlled to activate or remain in the first mode or a different stability threshold.

3. Device according to claim 2,
wherein the processing unit (22) is configured to determine the stability of the periodicity of the at least one PPG signal by determining the number of extrema for a measurement duration and determining a mean or average value and/or standard deviation of the number of extrema, in particular by determining a mean or average value and/or standard deviation of the number of extrema for two or more measurement periods and comparing the determined mean or average values and/or standard deviation determined for the two or more measurement periods.

4. Device according to claim 2 or 3,

wherein the processing unit (22) is configured to determine the signal quality, in particular the signal-to-noise ratio, of the at least one PPG signal; and
wherein the control unit (23) is configured to control the pulse oximeter (10) to

- activate or remain in the first mode, if the periodicity of the at least one PPG signal is stable, in particular if the determined stability is equal to or higher than a stability threshold, and the signal quality is high, in particular if the determined signal quality is equal to or higher than a quality threshold; or
- activate or remain in a second mode, if the stability of the at least one PPG signal is not stable, in particular if the determined stability is lower than the stability threshold, and/or the signal quality is low, in particular if the determined signal quality is lower than the quality threshold used for determining if the pulse oximeter (10) shall be controlled to activate or remain in the first mode or a different quality threshold.

5. Device according to any preceding claim,
wherein the processing unit (22) is configured to generate the control signal that,

i) when the at least one PPG signal is approaching one of its extreme points, controls the light detector to acquire a limited number of samples, in particular one or more samples below the maximum number of samples or preferably below half of the maximum number of samples, of the one or more PPG signals around the extreme point, and,
ii) when the at least one PPG signal has passed the extreme point, controls the light detector not to acquire samples of the one or more PPG signals in the one or more wavelength channels.

6. Device according to any preceding claim,
wherein the processing unit (22) is configured to detect if the at least one PPG signal is approaching one of its extreme points based on an evaluation of the recent signal course of the at least one PPG signal and/or the derivation or slope of the at least one PPG signal.

7. Device according to claim 6,
wherein the processing unit (22) is configured to determine that the at least one PPG signal is approaching one of its extreme points if

i) the absolute value of the difference between the actual signal value of the first PPG signal and the average value of the first PPG signal exceeds a value in the range of 25% to 45%, in particular that exceeds 30%, 40% or 45% of the difference between the last two extreme points or an average of the last differences between respective two consecutive extreme points, and/or
ii) if the derivation or slope falls below a value in the range of 25% to 50%, in particular that falls below 50% or 40% or 30% of the derivative of the last extreme point or an average of derivates of the last two or more extreme points.

8. Device according to any preceding claim,
wherein the processing unit (22) is configured to detect if the at least one PPG signal is approaching one of its extreme points based on physiological knowledge about the subject and/or the recent computation of the vital sign, in particular if a time period from the last extreme point has passed that corresponds to a value in the range of 90% to 99%, in particular 90% or 95% or 97,5%, of the time period between the last two extreme points or an average of the last multiple time periods between respective two consecutive extreme points.

9. Device according to any preceding claim,
wherein the processing unit (22) is configured to determine that the at least one PPG signal has passed the extreme point

i) if the absolute value of the difference between the actual signal value of the at least one PPG signal and the average value of the at least one PPG signal falls below a value in the range of 25% to 45%, in particular that falls below 30%, 40% or 45% of the difference between the last two extreme points or an average of the last multiple differences between respective two consecutive extreme points, and/or

ii) after a time period from reaching the extreme point that corresponds to the time period from activating the light emission in one or more wavelength ranges up to reaching the extreme point.

10. Device according to any preceding claim, wherein the control unit (23) is configured to activate and deactivate light emission in the one or more wavelength ranges for a time duration in the range up to 30% of the time period between the last two extreme points or an average of the last multiple time periods between respective two consecutive extreme points.

11. Device according to any preceding claim, wherein the control unit (23) is configured to control the light detector to activate and deactivate the light detection and/or generation of PPG signals in the one or more wavelength channels simultaneously or substantially simultaneously with activation and deactivation of the light emitter, in particular to activate and deactivate signal acquisition circuitry and/or signal processing circuitry of the light detector.

12. Device according to any preceding claim, wherein the processing unit (22) is configured to generate the vital signal, in particular blood oxygen saturation or heart rate or a modification of hemoglobin, from the extreme values of the at least two PPG signals.

13. System (1, 2) comprising:

- a pulse oximeter (10) comprising a light emitter (11) configured to emit light in one or more wavelength ranges and a light detector (12) configured to detect light in one or more wavelength channels reflected from skin of a subject or transmitted through tissue of the subject in response to irradiation by the light emitter, each wavelength channel covering one of the one or more wavelength ranges, and to generate one or more photoplethysmography, PPG, signals from the detected light in the one or more wavelength channels; and
- a device (20) for operating the pulse oximeter according to any preceding claim.

14. Method for operating a pulse oximeter (10) comprising a light emitter (11) configured to emit light in one or more wavelength ranges and a light detector (12) configured to detect light in one or more wavelength channels reflected from skin of a subject or transmitted through tissue of the subject in response to irradiation by the light emitter, each wavelength channel covering one of the one or more wavelength ranges, and to generate one or more photoplethysmography, PPG, signals from the detected light in the one or more wavelength channels, the method comprising:

- determining the periodicity of at least one PPG signal;
- predicting, based on the determined periodicity, if the at least one PPG signal is approaching one of its extreme points;
- computing a vital sign from the at least two PPG signals; and
- generating a control signal that,

i) when the at least one PPG signal is approaching one of its extreme points, controls the light emitter to activate light emission in the one or more wavelength ranges and/or controls the light detector to activate the light detection and/or generation of PPG signals in the one or more wavelength channels, and,

ii) when the at least one PPG signal has passed the extreme point, controls the light emitter to deactivate light emission in the one or more wavelength ranges and/or controls the light detector to deactivate the light detection and/or the generation of PPG signals in the one or more wavelength channels.

15. Computer program comprising program code means for causing a computer to carry out the steps of the method as claimed in claim 14 when said computer program is carried out on the computer.

FIG.1

FIG.2

FIG.3

FIG.4

FIG.5

FIG.6

FIG.7

100

| DETERMINE PERIODICITY | 101 |
| DETECT EXTREME POINT | 102 |
| GENERATE CONTROL SIGNAL | 103 |
| COMPUTE VITAL SIGN | 104 |

## FIG.8

## FIG.9

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/135692 A1 (LISOGURSKI DANIEL [US] ET AL) 19 May 2016 (2016-05-19) * paragraphs [0105] - [0107]; figure 4 * * paragraphs [0124] - [0130]; figure 9 * * paragraphs [0137] - [0138]; figure 12 * ----- | 1,5-15 | INV. A61B5/024 A61B5/1455 ADD. A61B5/00 |
| X | US 5 924 979 A (SWEDLOW DAVID B [US] ET AL) 20 July 1999 (1999-07-20) * column 2, line 65 - column 4, line 13 * * column 5, line 58 - column 7, line 8 * * column 8, lines 27-59 * ----- | 1-3,5-15 | |
| X | WO 2015/159187 A1 (INDIAN INST SCIENT [IN]) 22 October 2015 (2015-10-22) * page 19, line 22 - page 22, line 13 * ----- | 1,4-6, 8-10, 12-15 | |
| X | US 2021/392446 A1 (LUNNER THOMAS [US] ET AL) 16 December 2021 (2021-12-16) * paragraph [0284] * ----- | 1,13,14 | |

TECHNICAL FIELDS SEARCHED (IPC)

A61B

The present search report has been drawn up for all claims

| Place of search | Date of completion of the search | Examiner |
|---|---|---|
| The Hague | 7 March 2024 | Loveniers, Kris |

EPO FORM 1503 03.82 (P04C01)

## ANNEX TO THE EUROPEAN SEARCH REPORT
## ON EUROPEAN PATENT APPLICATION NO.

EP 23 20 1531

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2024

| Patent document cited in search report | | | Publication date | Patent family member(s) | | Publication date |
|---|---|---|---|---|---|---|
| US 2016135692 | A1 | | 19-05-2016 | EP | 2854640 A1 | 08-04-2015 |
| | | | | US | 2013324855 A1 | 05-12-2013 |
| | | | | US | 2016135692 A1 | 19-05-2016 |
| | | | | WO | 2013181377 A1 | 05-12-2013 |
| US 5924979 | A | | 20-07-1999 | AU | 1849697 A | 28-08-1997 |
| | | | | DE | 69700622 T2 | 08-06-2000 |
| | | | | EP | 0879014 A1 | 25-11-1998 |
| | | | | JP | 3817586 B2 | 06-09-2006 |
| | | | | JP | 2000504599 A | 18-04-2000 |
| | | | | US | 5746697 A | 05-05-1998 |
| | | | | US | 5924979 A | 20-07-1999 |
| | | | | WO | 9728739 A1 | 14-08-1997 |
| WO 2015159187 | A1 | | 22-10-2015 | NONE | | |
| US 2021392446 | A1 | | 16-12-2021 | CN | 113691917 A | 23-11-2021 |
| | | | | EP | 3913929 A1 | 24-11-2021 |
| | | | | US | 11134351 B1 | 28-09-2021 |
| | | | | US | 2021392446 A1 | 16-12-2021 |
| | | | | US | 2023412990 A1 | 21-12-2023 |

EPO FORM P0459

For more details about this annex : see Official Journal of the European Patent Office, No. 12/82